# EUROPEAN PATENT APPLICATION

(11) **EP 4 497 442 A1**
(43) Date of publication of application: **29.01.2025**
(21) Application number: 23188454.5
(22) Date of filing: 28.07.2023
(51) Int. Cl.: A61K 36/282, A61P 17/00, A61P 31/00, A61K 31/015, A61K 31/12

(54) **ANTI-DANDRUFF ACTIVITY OF ARTEMISIA VULGARIS ESSENTIAL OIL**

(71) Applicant: Centre for Aromatic Plants, Dehradun Uttarakhand 248011 (IN)
(72) Inventor: Chauhan, Nirpendra K., 248011 Dehradun (IN); Chauhan, Anil Kumar, 248011 Dehradun (IN); Kevlani, Nikita, 248011 Dehradun (IN); Naik, Gaurav, 248011 Dehradun (IN); Sah, Sunil, 248011 Dehradun (IN); Lohani, Hema, 248011 Dehradun (IN)
(74) Representative: Di Bernardo, Antonio

(57) **Abstract**

The present disclosure relates to the use of essential oil or an active fraction thereof from *Artemisia vulgaris* in the prevention and/or treatment of dandruff. The essential oil or an active fraction thereof from *Artemisia vulgaris* has an anti-fungal activity against *Malassezia furfur* and *Candida albicans.* Further, the present disclosure provides a composition comprising an essential oil or an active fraction thereof of *A. vulgaris.*

## Description

### FIELD OF THE INVENTION

The present disclosure relates to essential oil of *Artemisia vulgaris* for improving skin conditions or treating skin infections caused by fungi. More particularly, the present disclosure relates to *Artemisia vulgaris* essential oil for use in preventing and/or treating a dandruff condition or candidiasis. The present disclosure relates to an essential oil or an active fraction thereof from *Artemisia vulgaris* having anti-fungal activity against *Malassezia furfur* and *Candida albicans.*

### BACKGROUND OF THE INVENTION

Dandruff is a non-inflammatory skin condition that causes the flaking of the scalp, and nearly half of the global population is affected by this condition irrespective of gender or ethnicity. Dandruff, as such, is neither a clinical condition nor contagious, however, it has a severe socio-psychological impact as one might look unhygienic with having a flaky scalp. Various etiological agents could cause dandruff, however, *Malassezia furfur,* a dimorphic, lipophilic and yeast like fungus is one the major pathogen responsible for causing dandruff. The growth of *M. furfur* is highly dependent on an exogenous source of lipids as they lack the gene that is responsible for fatty acid synthesis. Therefore, the prevalence of *M. furfur* is usually found in but not limited to the seborrheic skin areas including face, scalp, and thorax.

Cutaneous candidiasis is another form of skin infection involved in superficial skin and mucous membranes, which includes intertrigo, diaper dermatitis, Erosio interdigitalis blastomycetica (EIB), perianal dermatitis, and candidal balanitis. There are various causative agents of cutaneous candidiasis, however, *Candida albicans* has been found to be predominantly involved in this disease. *C. albicans* has been categorized polymorphic fungus, which means it shows different morphological features such as ovoid-shaped budding yeast, elongated ellipsoid cells with pseudohyphae and/or as parallel-walled true hyphae. Moreover, these fungi have the ability to survive and grow in different pH, which makes them more virulent than the other fungus groups. Meanwhile, the occurrence of candidiasis has been observed more frequently due to an increase in the number of immunocompromised patients in recent years, which poses a severe threat as well as challenges to developing new anti- *C. albicans* agents.

Many anti-dandruff and anti-candidiasis treatments and several drugs have been developed. In the case of dandruff, for example, zinc pyrithione, ciclopirox, selenium sulfide, minoxidil, coal tar, and ketoconazole are available in the form of shampoo, gels and solutions in the market. However, these agents exhibit significant toxicity to the skin cells, causing a dry scalp and discoloration of hair, ketoconazole, in particular, has been reported to have toxicity on human cells and cause urticaria, a skin reaction that causes itchy welts.

Moreover, besides the side effects of these antifungal drugs, another problem that arises is resistance of the fungus against these drugs, for instance, Candida species have been reported to have developed resistance against azoles and echinocandins which is an alarming situation. Therefore, the research of the present time has shifted towards the development of alternative anti-fungal candidates from natural resources, which can ensure the clearance of fungus along with no toxicity to the skin.

*Artemisia vulgaris,* commonly known as 'mugwort' has significant importance in ancient medical science and has been termed a "mother herb" for its various medicinal uses. The herb is found to be located nearly all over the world, however, the biological activity of the herb is associated with its demography. In recent years, a number of studies have been carried out that explore the scientific basis behind its use in traditional setups. For example, a study carried out by Khan, K.A. A, 2015, showed that the root of *A. vulgaris* has potent antihyperlipidemic activity in a hyperlipidaemic rat. Biological activities of *A. vulgaris,* including hepatoprotective, anti-spasmolytic, analgesic, estrogenic, cytotoxic, antibacterial, antifungal, hypotensive, and broncholytic effects, have also been reported.

The essential oil of *A. vulgaris* contains bioactive chemical constituents and has been shown to possess beneficial effects. Further, a wide range of variations in the chemical composition of *A. vulgaris* essential oil has been observed in various reported studies. For example, a study carried out by Dianna et al. reported that 1,8-Cineol is the major compound of *A. vulgaris.* Misra, L.N., 1986, discloses that α-Thujone is the major compound found in the commercial *A. vulgaris* essential oil. Malik S, et al., 2019, reported that caryophyllene was found to be the major compound in *A. vulgaris* essential oil. Therefore, this variation in the chemical composition of the *A. vulgaris* essential oil might be attributed to its different chemotypes.

The present disclosure is designed to solve the technical problems present in the art, excellent anti-dandruff and anti-candidiasis activity against *M. furfur* and *C. albicans* with no side effects on the skin.

In order to solve the above technical problem, the inventors of the present disclosure evaluated the anti-dandruff and anti-candidiasis activity of *A. vulgaris* essential oil and also found that artemisia ketone is the major component.

### SUMMARY OF THE INVENTION

This summary is provided to introduce a selection of concepts in a simplified format that are further described in the detailed description of the invention.

In one aspect of the present disclosure, there is provided an essential oil or an active fraction thereof from *Artemisia vulgaris* for use in preventing and/or treating a dandruff condition.

In an embodiment of the present disclosure, there is provided an essential oil or an active fraction thereof from *Artemisia vulgaris* for use in preventing and/or treating a dandruff condition, wherein the dandruff condition is caused by *Malassezia furfur.*

In an embodiment of the present disclosure, there is provided an essential oil or an active fraction thereof from *Artemisia vulgaris* for use in preventing and/or treating a dandruff condition, wherein the essential oil comprises artemisia ketone in an amount of 20-27% by weight of the total essential oil.

In an embodiment of the present disclosure, there is provided an essential oil or an active fraction thereof from *Artemisia vulgaris* for use in preventing and/or treating a dandruff condition, wherein the essential oil comprises:
artemisia ketone in an amount of 20-27% by weight of the total essential oil;
trans-caryophyllene in an amount of 7-11% by weight of the total essential oil;
acoradeine in an amount of 2-7% by weight of the total essential oil; and
beta-himachaiene in an amount of 2-6% by weight of the total essential oil.

In an embodiment of the present disclosure, there is provided an essential oil or an active fraction thereof from *Artemisia vulgaris* for use in preventing and/or treating a dandruff condition, wherein the active fraction is artemisia ketone.

In one aspect of the present disclosure, there is provided a composition, comprising:
an essential oil or an active fraction thereof from *Artemisia vulgaris;* and
at least one pharmaceutically acceptable carrier, diluent or excipient for use in preventing and/or treating a dandruff condition.

In an embodiment of the present disclosure, there is provided a composition, wherein the dandruff condition is caused by *Malassezia furfur.*

In an embodiment of the present disclosure, there is provided a composition, wherein the active fraction is artemisia ketone.

In an embodiment of the present disclosure, there is provided use of an *Artemisia vulgaris* essential oil or an active fraction thereof for the manufacture of a medicament for preventing or treating infection caused by *Malassezia furfur.*

In an embodiment of the present disclosure, there is provided use of an *Artemisia vulgaris* essential oil or an active fraction thereof for the manufacture of a medicament for preventing or treating infection caused by *Malassezia furfur,* wherein the active fraction is artemisia ketone.

In an embodiment of the present disclosure, there is provided an essential oil or an active fraction thereof from *Artemisia vulgaris* for use in preventing and/or treating candidiasis.

In an embodiment of the present disclosure, there is provided an essential oil or an active fraction thereof from *Artemisia vulgaris* for use in preventing and/or treating candidiasis, wherein the candidiasis is caused by *Candida albicans.*

In an embodiment of the present disclosure, there is provided an essential oil or an active fraction thereof from *Artemisia vulgaris* for use in preventing and/or treating candidiasis, wherein the candidiasis is cutaneous candidiasis.

In an embodiment of the present disclosure, there is provided an essential oil or an active fraction thereof from *Artemisia vulgaris* for use in preventing and/or treating candidiasis, wherein the active fraction is artemisia ketone.

In an embodiment of the present disclosure, there is provided an essential oil or an active fraction thereof from *Artemisia vulgaris* for use in preventing and/or treating candidiasis, wherein the essential oil comprises artemisia ketone in an amount of 20-27% by weight of the total essential oil.

### BRIEF DESCRIPTION OF THE DRAWINGS

The following drawings form part of the present specification and are included to further demonstrate certain aspects of the present disclosure, the inventions of which can be better understood by reference to one or more of these drawings in combination with the detailed description of specific embodiments presented herein.
Figure 1 illustrates the chemical profiling of *Artemisia vulgaris* essential oil analysed by GC-MS.
Figure 2 illustrates the purity of artemisia ketone in fractionated essential oil samples analysed by GC-MS
Figure 3 illustrates the anti-dandruff efficacy of *A. vulgaris* essential oil and artemisia ketone against *M. furfur,* determined by the zone of inhibition assay. Ketoconazole was used as a positive control.
Figure 4 illustrates the anti-dandruff efficacy of *A. vulgaris* essential oil and artemisia ketone against *C. albicans,* determined by the zone of inhibition assay. Ketoconazole was used as a positive control.

### DETAILED DESCRIPTION OF THE INVENTION

Those skilled in the art will be aware that the present disclosure is subject to variations and modifications other than those specifically described. It is to be understood that the present disclosure includes all such variations and modifications. The disclosure also includes all such steps of the process, features of the product, referred to or indicated in this specification, individually or collectively, and any and all combinations of any or more of such steps or features.

### Definitions

For convenience, before further description of the present disclosure, certain terms employed in the specification, and examples are collected here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The term "including" is used to mean "including but not limited to". "Including" and "including but not limited to" are used interchangeably.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods, and materials are now described. All publications mentioned herein are incorporated herein by reference.

The present disclosure is not to be limited in scope by the specific embodiments described herein, which are intended for the purposes of exemplification only. Functionally equivalent products and methods are clearly within the scope of the disclosure, as described herein.

The inventors of the present disclosure have evaluated the chemical composition of *Artemisia vulgaris* essential oil and its efficacy against *Malassezia furfur* and *Candida albicans.* The results of the GC-MS analysis demonstrated that artemisia ketone was the major component in the essential oil extracted from *A. vulgaris.* Moreover, in order to compare the antifungal activity of *A. vulgaris* oil and its major compound, artemisia ketone, the inventors fractionated the artemisia ketone up to 92% purity from the *A. vulgaris* oil. Further, the inventors performed the zone of inhibition (ZOI), minimum inhibitory concentration (MIC) and minimum fungicidal concentration (MFC) assay of the *A. vulgaris* oil and ketone against *M. furfur* and *C. albicans.* The results obtained suggested that the artemisia ketone was more active against both fungal strains compared to *A. vulgaris* oil. Also, a two-fold reduction in MIC and MFC value was observed in artemisia ketone fraction compared to *A. vulgaris* oil against *C. albicans.* Artemisia ketone was significantly more effective against *M. furfur* compared to *A. vulgaris* essential oil. Thus, the results of the present disclosure demonstrated that the *A. vulgaris* oil and artemisia ketone both have potent cytotoxicity against *M. furfur* and *C. albicans,* however, artemisia ketone has shown better activity than the *A. vulgaris essential* oil.

In one aspect of the present disclosure, there is provided an essential oil or an active fraction thereof from *Artemisia vulgaris* for use in preventing and/or treating a dandruff condition.

In an embodiment of the present disclosure, there is provided an essential oil or an active fraction thereof from *Artemisia vulgaris* for use in preventing and/or treating a dandruff condition, wherein the dandruff condition is caused by *Malassezia furfur.*

In an embodiment of the present disclosure, there is provided an essential oil or an active fraction thereof from *Artemisia vulgaris* for use in preventing and/or treating a dandruff condition, wherein the essential oil comprises artemisia ketone in an amount of 20-27% by weight of the total essential oil.

In an embodiment of the present disclosure, there is provided an essential oil or an active fraction thereof from *Artemisia vulgaris* for use in preventing and/or treating a dandruff condition, wherein the essential oil comprises:
artemisia ketone in an amount of 20-27% by weight of the total essential oil;
trans-caryophyllene in an amount of 7-11% by weight of the total essential oil;
acoradeine in an amount of 2-7% by weight of the total essential oil; and
beta-himachaiene in an amount of 2-6% by weight of the total essential oil.

In an embodiment of the present disclosure, there is provided an essential oil or an active fraction thereof from *Artemisia vulgaris* for use in preventing and/or treating a dandruff condition, wherein the active fraction is artemisia ketone.

In one aspect of the present disclosure, there is provided a composition, comprising:
an essential oil or an active fraction thereof from *Artemisia vulgaris;* and
at least one pharmaceutically acceptable carrier, diluent or excipient for use in preventing and/or treating a dandruff condition.

In an embodiment of the present disclosure, there is provided a composition, wherein the dandruff condition is caused by *Malassezia furfur.*

In an embodiment of the present disclosure, there is provided a composition, wherein the active fraction is artemisia ketone.

In an embodiment of the present disclosure, there is provided use of an *Artemisia vulgaris* essential oil or an active fraction thereof for the manufacture of a medicament for preventing or treating infection caused by *Malassezia furfur.*

In an embodiment of the present disclosure, there is provided use of an *Artemisia vulgaris* essential oil or an active fraction thereof for the manufacture of a medicament for preventing or treating infection caused by *Malassezia furfur,* wherein the active fraction is artemisia ketone.

In an embodiment of the present disclosure, there is provided an essential oil or an active fraction thereof from *Artemisia vulgaris* for use in preventing and/or treating candidiasis.

In an embodiment of the present disclosure, there is provided an essential oil or an active fraction thereof from *Artemisia vulgaris* for use in preventing and/or treating candidiasis, wherein the candidiasis is caused by *Candida albicans.*

In an embodiment of the present disclosure, there is provided an essential oil or an active fraction thereof from *Artemisia vulgaris* for use in preventing and/or treating candidiasis, wherein the candidiasis is cutaneous candidiasis.

In an embodiment of the present disclosure, there is provided an essential oil or an active fraction thereof from *Artemisia vulgaris* for use in preventing and/or treating candidiasis, wherein the active fraction is artemisia ketone.

In an embodiment of the present disclosure, there is provided an essential oil or an active fraction thereof from *Artemisia vulgaris* for use in preventing and/or treating candidiasis, wherein the essential oil comprises artemisia ketone in an amount of 20-27% by weight of the total essential oil.

### EXAMPLES

### Chemicals and Reagents

For inoculation, *Malassezia furfur* (MTCC 1374) and *Candida albicans* (MTCC 3017) were Procured from Microbial Type Culture Collection (MTCC) Chandigarh. Sabouraud Dextrose Agar (SDA), Sabouraud Dextrose Broth (SDB) and Yeast Extract Peptone Dextrose Agar (YEPD) and Yeast Extract Peptone Dextrose Broth Media were Procured from HiMedia. Maize Corn Oil, DMSO and Methanol were Procured from Sigma-Aldrich USA, and the antifungal drug amphotericin B and ketoconazole which were used as standard procured from Sigma-Aldrich USA.

### Fungal Strain and Culture

The fungal strain of *Malassezia furfur* and *Candida albicans* were obtained from the Microbial Type Culture Collection (MTCC), India. Sabouraud Dextrose Agar (HiMedia, India) along with 2% olive oil was used to culture *M. furfur* and YPD agar was used for *C. albicans.* Briefly, *M. furfur* culture were streaked in SDA (2% Maize corn oil) plates and incubated for 7 days at 37°C. After incubation colonies were picked and inoculated in SDB (2% Maize Corn oil) broth and further incubation of 72 hours was given and this culture was used for the experiment. On the other hand, *C. albicans* were cultured in YEPD agar for 24 to 48 hours. The fresh colonies were then inoculated in YEPD broth medium, followed by further incubation for 24-48 hours and this culture was used for the experiment.

### Collection of Essential Oil of Artemisia vulgaris Plant

The essential oil of *A. vulgaris* plant was collected from the Uttarakhand, India.

### Fractionation of Artemisia Ketone from Essential Oil

Volatile fractions were obtained from *A. vulgaris* essential oil.

### Gas Chromatography and Mass Spectrophotometry Analysis

GC-MS analysis of the oil was performed with a Perkin Elmer Claurs 500 gas chromatography equipped with a split / splitless injector (split ratio 50:1) data handling system. The column was Rtx5 capillary column (60 m × 0.32 mm, 0.25 µm film thickness). Helium (He) was the carrier gas at a flow rate of 1.0 mL/min. The GC was interfaced with (Perkin Elmer Clarus 500) mass detector operating in the EI positive mode. The mass spectra were generally recorded over m/z 40-500 amu that revealed the total ion current (TIC) chromatograms. The temperature program was used the same as described above for GC analysis. The temperature of the injector, transfer line and ion source were maintained at 210°C, 210°C, and 200°C, respectively. Identification of the components was made on the basis of retention time, and retention indices, determined with reference to homologous series of n-alkanes (C8 -C24 Sigma-Aldrich) under identical experimental conditions, co-injection with authentic standard compounds, mass spectra with the library provided by instrument software (NIST/Wiley) and by comparing their mass spectra with those reported in literature 20. Quantification of each compound was performed on the basis of their GC peak area, using the normalization procedure without using correction factors.

### Determination of Zone of Inhibition

To determine the zone of inhibition an agar well diffusion assay was carried out. Briefly, 100µl of fresh *M. furfur* culture broth was inoculated in the SDA agar and wells of 6mm diameter were created using a sterile cork-borer. 50µl of 50% *A. vulgaris* essential oil and artemisia ketone diluted in Tween 20 and MeOH were placed in the well and diluent was placed in the control well. Also, ketoconazole was used as a positive control. Plates were then incubated at 37°C in an incubator (Brunswick Innova 42) for 96 hours. Similarly, 100µl of *C. albicans* culture (OD-0.5) was inoculated on YPD agar plates followed by the well preparation. Treatment of essential oil and artemisia ketone was done as described above, however, amphotericin B was used as a positive control in the case of *C*. *albicans.* Plates were incubated at 37°C for 24 hours and after the incubation period the zone of inhibition was measured in mm.

### Determination of Minimum Inhibitory Concentration

To determine the minimum inhibitory concentration a broth dilution assay was carried out. Briefly, 100µl of *A. vulgaris* essential oil and artemisia ketone were placed in 96 well plate in a serial dilution manner. After that 100µl fresh *M. furfur* and/or *C. albicans* culture broth (OD-0.5) was inoculated in the wells and was mixed by pipetting. Plates were then incubated at 37°C in an incubator (Brunswick Innova 42) for 72 hours in the case of *M. furfur* and 24 hours for *C. albicans.* Once the incubation was completed the wells were visualized for growth. The lowest concentration that has no visual growth was considered as the minimum inhibitory concentration.

### Determination of Minimum Fungicidal Concentration

Determination of minimum fungicidal concentration was carried out in similar to minimum inhibitory concentration. However, in the case of MFC, *M. furfur* and *C. albicans* treated with *A. vulgaris* essential oil and artemisia ketone were plated on Sabouraud Dextrose Agar and Yeast Potato Dextrose agar and incubated for 72 hours and 24 hours, respectively. After, the incubation period, the plates were observed for the fungal colonies and the concentration that had no colonies were considered as the minimum fungicidal concentration of *A. vulgaris* essential oil and artemisia ketone against *M. furfur* and *C. albicans.*

### Statistical Analysis

All the samples were analysed in triplicate. Experimental results were depicted as the mean ± standard error of the mean (SEM). Statistical significance was calculated using the Students t-tests. * represents P< 0.05, ** represents P< 0.01, and *** represents P< 0.001.

### Results

### Chemical composition of A. vulgaris essential oil

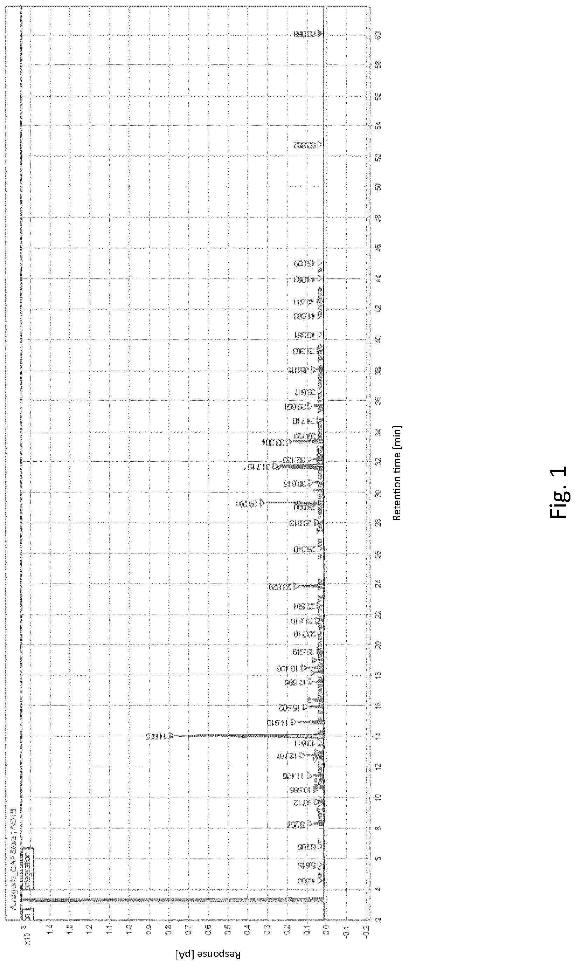

The GC-MS analysis was carried out to examine the chemical composition of *A. vulgaris* essential oil. The data demonstrated that artemisia ketone was the major compound in *A. vulgaris* essential oil (25.62%) followed by trans-caryophyllene (9.77%), acoradeine (6.77%), beta-himachaiene (4.95), 1,8 Cineole (2.29%), Borneol (2.34) etc. as depicted in Table 1 and Figure 1.

**Table 1 presents the major chemical constituent of A. vulgaris essential oil analyzed by GC-MS.**

| **Sr. No.** | **Components** | **RT** | **Area%** |
|---|---|---|---|
| 1 | Artemisia Ketone | 14.03 | 25.62 |
| 2 | Trans-caryophyllene | 29.29 | 9.77 |
| 3 | Acoradeine>10-epi-beta< | 31.71 | 6.77 |
| 4 | Beta-Himachaiene | 33.30 | 4.95 |
| 5 | 1,8 Cineole | 12.79 | 2.29 |
| 6 | Borneol | 18.50 | 2.34 |
| 7 | Camphor | 17.58 | 1.24 |
| a | Beta-Thujone | 15.90 | 1.98 |
| 9 | 4-Terpineol | 18.99 | 0.89 |
| 10 | P-cymene | 12.50 | 0.64 |
| 11 | Camphene | 9.71 | 0.58 |
| 12 | D-Limonene | 12.79 | 0.32 |
| 13 | Bornyl acetate | 23.66 | 0.23 |
| 14 | Alpha-Thujone | 15.56 | 0.21 |
| 15 | Chry santhenyl acetate | 22.59 | 0.12 |

### Determination of purity of fractionated artemisia ketone

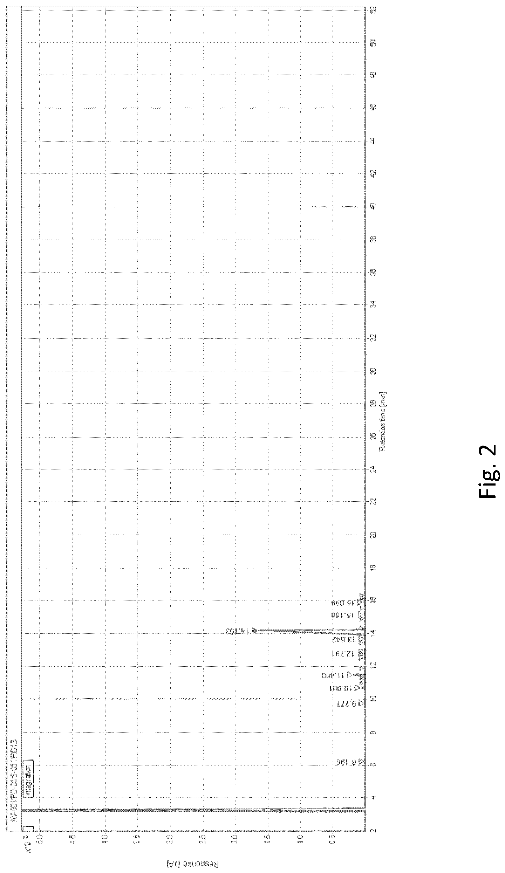

Artemisia ketone was fractionated from *Artemisia vulgaris* essential oil through fractional distillation, and the purity of artemisia ketone was identified by GC-MS analysis. In the present disclosure, the data revealed that the fractionated sample contained 91.37% purity of artemisia ketone (Figure 2).

### Zone of inhibition of A. vulgaris oil and artemisia ketone against M. furfur

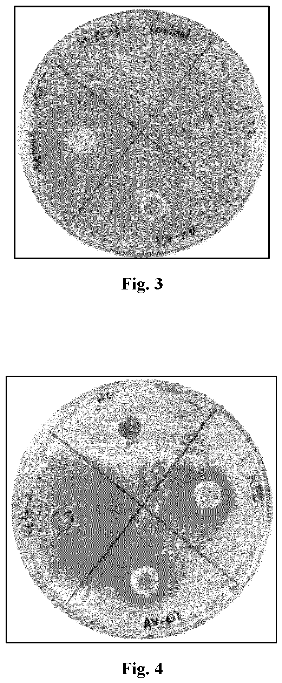

To determine the antifungal efficacy of *A. vulgaris* essential oil and artemisia ketone zone of inhibition assay was done. As depicted in Figure 3 and

Table 2, *A. vulgaris* essential oil demonstrated a significant zone of inhibition against *M. furfur* i.e., 25 ± 1.5 mm. On the other hand, artemisia ketone exhibited 35 ± 1.8 mm of zone of inhibition against *M. furfur* which was far better than the ketoconazole which showed a zone of inhibition of 29 ± 2.0 mm of zone.

**Table 2 presents the anti-dandruff efficacy of A. vulgaris essential oil and artemisia ketone against M. furfur, determined by the zone of inhibition assay.**

| **Sample** | **Zone of Inhibition (mm)** |
|---|---|
| Control | 0 |
| *Artemisia vulgaris* Essential Oil | 25 ± 1.5 |
| Artemisia Ketone | 35 ± 1.8 |
| Ketoconazole | 29 ± 2.0 |

### Minimum inhibitory concentration and minimum fungicidal concentration of A. vulgaris oil and artemisia ketone against M. furfur

In addition to the zone of inhibition, the MIC and MFC values of *A. vulgaris* essential oil and artemisia ketone against *M. furfur* were determined. The result suggested that 10 mg/ml was the MIC value of *A. vulgaris* essential oil and 20 mg/ml was found to be the MFC value against *M*. *furfur.* However, artemisia ketone showed much better activity than *A. vulgaris* essential oil exhibited 7.5 mg/ml as a MIC value and 15 mg/ml was calculated as a MFC value against *M. furfur* (Table 3).

**Table 3 presents the minimum inhibitory concentration (MIC) and minimum fungicidal (MFC) of A. vulgaris essential oil and artemisia ketone against M. furfur.**

| **Sample** | **MIC (mg/ml)** | **MFC (mg/ml)** |
|---|---|---|
| *Artemisia vulgaris* essential oil | 10 | 20 |
| Ketone | 7.5 | 15 |

### Zone of inhibition of A. vulgaris oil and artemisia ketone against C. albicans

In order to determine the ability of *A. vulgaris* essential oil and artemisia ketone to inhibit the growth of *C. albicans* the zone of inhibition assay was performed. However, the results suggested that all the test samples exhibited significant activity in the case of *C*. *albicans* where, *A. vulgaris* essential oil showed 29 ± 1.15 mm of zone and artemisia ketone showed 35 ± 0.6 mm of zone of inhibition at a concentration of 20 mg/ml which was comparable with ketoconazole which showed 22 ± 1.5 mm zone (Table 4).

**Table 4 presents the anti-dandruff efficacy of A. vulgaris essential oil and artemisia ketone against C. albicans, determined by the zone of inhibition assay.**

| **Sample** | **Zone of Inhibition (mm)** |
|---|---|
| Control | 0 |
| *Artemisia vulgaris* Essential Oil | 29 ± 1.15 |
| Artemisia Ketone | 38 ± 0.6 |
| Ketoconazole | 33 ± 2.0 |

### Minimum inhibitory concentration and minimum fungicidal concentration of A. vulgaris oil and artemisia ketone against C. albicans

Furthermore, the MIC and MFC *of A. vulgaris* essential oil and artemisia ketone against C. *albicans* were evaluated. As depicted in Table 5, the MIC value *of A. vulgaris* essential oil was observed to be 10 mg/ml and the MFC value was 20 mg/ml. On the other hand, artemisia ketone exhibited significantly better activity than essential oil. Artemisia ketone exhibited 5 mg/ml as the MIC value and 10 mg/ml as the MFC value suggesting two times better activity than the essential oil alone (Table 5).

**Table 5 presents the minimum inhibitory concentration (MIC) and minimum fungicidal (MFC) of A. vulgaris essential oil and artemisia ketone against C. albicans.**

| **Sample** | **MIC (mg/ml)** | **MFC (mg/ml)** |
|---|---|---|
| *Artemisia vulgaris* essential oil | 10 | 20 |
| Ketone | 5 | 10 |

The present disclosure demonstrated the anti-dandruff and anti-candidiasis activity of *A. vulgaris* essential oil and its active fraction i.e., artemisia ketone. Further, the present disclosure demonstrated that the essential oil of *A. vulgaris* is rich in artemisia ketones. Moreover, artemisia ketone is responsible for the anti-dandruff and anti-candida activity of *A. vulgaris* essential oil, which helps in the formulation of anti-dandruff shampoo or gel in order to treat dandruff and cutaneous candidiasis.

It will thus be seen that the objects set forth above, among those made apparent from the preceding description, are efficiently attained, and since certain changes may be made in the constructions set forth without departing from the spirit and scope of the invention, it is intended that all matter contained in the above description shall be interpreted as illustrative and not in a limiting sense. The invention has been described with reference to preferred and alternate embodiments. Modifications and alterations will become apparent to those skilled in the art upon reading and understanding the detailed discussion of the invention provided herein. This invention is intended to include all such modifications and alterations insofar as they come within the scope of the present invention. These and other modifications of the preferred embodiments as well as other embodiments of the invention will be obvious from the disclosure herein, whereby the foregoing descriptive matter is to be interpreted merely as illustrative of the invention and not as a limitation.

Finally, to the extent necessary to understand or complete the disclosure of the present invention, all publications, patents, and patent applications mentioned herein are expressly incorporated by reference therein to the same extent as though each were individually so incorporated.

## Claims

1. An essential oil or an active fraction thereof from *Artemisia vulgaris* for use in preventing and/or treating a dandruff condition.

2. The essential oil or an active fraction thereof for use according to claim 1, wherein the dandruff condition is caused by *Malassezia furfur.*

3. The essential oil or an active fraction thereof for use according to claims 1-2, wherein the essential oil comprises:
artemisia ketone in an amount of 20-27% by weight of the total essential oil;
trans-caryophyllene in an amount of 7-11% by weight of the total essential oil;
acoradeine in an amount of 2-7% by weight of the total essential oil; and
beta-himachaiene in an amount of 2-6% by weight of the total essential oil.

4. The essential oil or an active fraction thereof for use according to claims 1-2, wherein the active fraction is artemisia ketone.

5. A composition, comprising:
an essential oil or an active fraction thereof from *Artemisia vulgaris;* and
at least one pharmaceutically acceptable carrier, diluent or excipient for use in preventing and/or treating a dandruff condition.

6. The composition according to claim 5, wherein the dandruff condition is caused by *Malassezia furfur.*

7. The composition according to claims 5-6, wherein the active fraction is artemisia ketone.

8. Use of an *Artemisia vulgaris* essential oil or an active fraction thereof for the manufacture of a medicament for preventing or treating infection caused by *Malassezia furfur.*

9. The use of the *Artemisia vulgaris* essential oil or an active fraction thereof according to claim 8, wherein the active fraction is artemisia ketone.

10. An essential oil or an active fraction thereof from *Artemisia vulgaris* for use in preventing and/or treating candidiasis.

11. The essential oil or an active fraction thereof for use according to claim 10, wherein the candidiasis is caused by *Candida albicans.*

12. The essential oil or an active fraction thereof for use according to claims 10-11, wherein the candidiasis is cutaneous candidiasis.

13. The essential oil or an active fraction thereof for use according to claims 10-12, wherein the active fraction is artemisia ketone.

14. The essential oil or an active fraction thereof for use according to claims 10-12, wherein the essential oil comprises artemisia ketone in an amount of 20-27% by weight of the total essential oil.
